# EUROPÄISCHE PATENTANMELDUNG

(11) **EP 4 830 771 A2**
(43) Veröffentlichungstag der Anmeldung: **28.10.2026**
(21) Anmeldenummer: 26203631.2
(22) Anmeldetag: 02.07.2018
(51) Int. Cl.: A61N 2/02

(54) **SYSTEM ZUR BEHANDLUNG MIT KERNSPINRESONANZEN**

(30) Priorität: 04.07.2017 DE 102017114856
(62) Dokumentnummer(n) der früheren Anmeldung(en) nach Art. 76 EPÜ: 18739793.0 / 3 648 840
(71) Anmelder: MedTec Medizintechnik GmbH, 35398 Gießen (DE)
(72) Erfinder: Muntermann, Axel, verstorben (DE); Egg, Margit, 6143 Matrei am Brenner (AT)
(74) Vertreter: Blumbach · Zinngrebe Patentanwälte PartG mbB

(57) **Zusammenfassung**

Die Erfindung betrifft ein Verfahren zur Einstellung eines Gerätes zur Behandlung mit Kernspinresonanzen. Dabei wird der Chronotyp des Benutzers ermittelt. Basierend darauf wird das Gerät eingestellt.

## Beschreibung

### Gebiet der Erfindung

Die Erfindung betrifft ein Verfahren zur Einstellung, der gezielten Kontrolle, Werteerfassung, Nachregulierung und insbesondere Eichung, eines Gerätes zur Behandlung mit Kernspinresonanzen. Weiter betrifft die Erfindung ein Gerät zur Behandlung mit Kernspinresonanzen, ein Behandlungssystem sowie die Verwendung von Kernspinresonanzen, insbesondere zu therapeutischen und kosmetischen Zwecken.

### Hintergrund der Erfindung

Die Verwendung von Kernspinresonanzgeräten zur Erzielung von therapeutischen und kosmetischen Wirkungen ist bekannt.

Die therapeutische Wirkung bei der Behandlung von menschlichem oder tierischem Gewebe mit Kernspinresonanzen ist bereits in der europäischen Patentschrift EP 1 089 792 B1 (Patentinhaber Axel Muntermann) beschrieben.

Bei dem unter der Handelsbezeichnung MBST^{®} betriebenen Therapiegeräten handelt es sich um Geräte, bei denen ein Magnetfeld mit homogener Feldstärke in der Behandlungszone von einem senkrecht eingestrahlten Wechselfeld überlagert wird, um in der Behandlungszone Kernspinresonanzen zu erzielen. Bei entsprechender Abstimmung der Frequenz des Wechselfeldes auf die Feldstärke des senkrecht stehenden homogenen Feldes können Kernspinresonanzen in der Behandlungszone erzeugt werden. Die Feldstärke des homogenen Magnetfeldes wird mit einer Modulationsfrequenz fₘ moduliert und setzt sich so aus einem Sockelbetrag und einem sich verändernden Modulationsbetrag zusammen. Aufgrund der Modulation wird bei jedem Durchlauf einmal die Larmor-Bedingung erreicht, so dass zyklisch Kernspinresonanzen im Behandlungsvolumen erzeugt werden.

Inhomogenitäten des statische Grundfeldes B₀ werden über den Weg der Adiabatic Fast Passage (AFP) ausgeglichen, die für die Umkehrung von Kernspin-Orientierungen sorgt.

Über eine in die Behandlungseinheit eingebrachte Empfängerspule mit elektronischer Überwachungselektronik kann die ordnungsgemäße Funktion des Kernspinbehandlungsfeldes, die Behandlungsparameter, Kompensation von beeinflussenden Störfaktoren wie Metalle im Behandlungsbereich, Veränderungen in der Feldstärke und Intensität der in den Körper eingebrachten Energiedosis, der benötigten Resonanzbedingungen und sonstige vorbestimmte wichtige Prüfparameter optisch und akustisch überwacht und für Nachregulierungsprozesse der Qualität, der Effektivität und der Sicherheit genutzt werden.

Es hat sich herausgestellt, dass mittels der Behandlung mit Kernspinresonanzen sowohl kosmetische, wie beispielsweise bei der Behandlung von Bindegewebeschwäche der Haut und Cellulite, als auch therapeutische Effekte in allen Bereichen gestörter oder degenerativer Zellfunktionen im Bereich der Gelenke des Knochens, der Organe, Bänder, Muskel, Sehnen sowohl in der nachoperativen Wundheilung, Hautverbrennungen, der Stoffwechsel- bzw. Durchblutungsstörung im Bereich des Knochens, der Fraktur-heilung, etc. als auch in anderen regenerationsfähigen Bereichen erzielt werden können.

Körpereigene Signale und elektrische Prozesse nehmen in der Wissenschaft einen immer größeren Stellenwert ein. Das ureigenste Signal ist zum Beispiel das EKG oder das EEG.

Studien zeigen, das piezoelektrische Prozesse im Bereich der Zellregeneration zwingend notwendig (ATP) sind, um gestörte Zellfunktion auszugleichen oder zu erhalten (Steinecker-Frohnwieser B, Weigl L, Weberhofer G, Kullich W, Kress HG. The Influence of Nuclear Magnetic Resonance Therapy (NMRT) and Interleukin IL1-b Stimulation on Cal 78 Chondrosarcoma Cells and C28/I2 Chondrocytes. J Orthopedics Rheumatol. 2014;1(3): 9).

Fehlen diese oder werden diese nur noch unzureichend erzeugt (fehlende Bewegungsenergie durch Druck- und Zugprozesse) stellen sich Degenerationseffekte ein, die durch eingekoppelte Kernspinresonanzfelder sehr effektiv und wirkungsvoll ausgeglichen werden können.

Grundlegend hierfür ist die Einhaltung der Übertragungswege in Resonanz bis zum Regenerationsort der ausgewählten Zellgruppe (Knorpel-, Knochen-, Haut-, Organzellen etc.)

Studien erbrachten den Nachweis, dass durch die Überwachungs- und Regelelektronik des Kernspinresonanzverfahrens der Sauerstoffgehalt (Hypoxie) und die ATP der Zellen nach Kernspinresonanzbehandlungen hochsignifikant gesteigert (siehe Steinecker-Frohnwieser et al.), je nach Steuermuster die Zellteilungsrate beschleunigt oder verlangsamt werden kann (N. Budny; Kom. Leiter Prof. Dr. med. D. Palmes, Prof. Dr. med. Dipl.-Ing. H.U. Spiegel; Inaugural-Dissertation zur Erlangung des doctor rerum medicinalium der Medizinischen Fakultät der Westfälischen Wilhelms-Universität Münster, Klinik für Allgemein- und Viszeralchirurgie, Abteilung Chirurgische Forschung, Universitätsklinikum Münster, Deutschland; Oktober 2015).

Ausschlaggeben für den gezielten Behandlungserfolg mit Kernspinresonanz ist präzise Ermittlung des Zellresonanzfensters der einzelnen zu regenerierenden Zellgruppen.

Unterschiedliche Zellgruppen haben unterschiedliche niederfrequente Resonanzansprechmuster, was bei Behandlungen mit Kernspinresonanz genutzt wird.

Ein Schwerpunkt derzeitiger Behandlungen mit Kernspinresonanzen sind Schmerzpatienten, welche unter degenerativen Veränderungen des Stütz- und Bewegungsapparates wie Gelenkarthrose, Osteoporose sowie Sport- und Unfallverletzung leiden.

So beschreibt beispielsweise der Artikel: KULLICH W., J. OVERBECK, H. U. SPIEGEL (2013): One-year-survey with multicenter data of more than 4,500 patients with degenerative rheumatic diseases treated with therapeutic nuclear magnetic resonance. J Back Musculoskelet Rehabil 26, 93-104, eine signifikante Verbesserung der Schmerzsymptomatiken bei Rheuma-Patienten durch die Behandlung mit der MBST^{®}-Kernspinresonanztherapie.

Die MBST^{®}-Therapiesysteme arbeiten mit geringeren Feldstärken als die für die Diagnose verwendeten bildgebenden Kernspinresonanz-Tomographen. Es hat sich aber überraschenderweise herausgestellt, dass gerade bei derartigen, mit niedrigen Feldstärken arbeitenden Systemen gute Behandlungserfolge erreichbar sind.

Es hat sich weiter herausgestellt, dass die Wirksamkeit der Behandlung u.a. auch von der Modulationsfrequenz des homogenen Feldes, der Anzahl der Behandlungen, der Behandlungsdauer und von den Intervallen, also dem Abstand zwischen mehreren Behandlungen, abhängig ist.

Je nach Indikation wurden derartige Parameter anfangs empirisch bestimmt, um optimale Behandlungsparameter zu ermitteln, zu bestätigen und somit die Wirksamkeit der MBST^{®}-Therapie weiter zu verbessern sowie die Indikationsbreite der Anwendung zu erweitern.

### Aufgabe der Erfindung

Der Erfindung liegt demgegenüber die Aufgabe zugrunde, ein Verfahren zur Einstellung, insbesondere der gezielten Kontrolle, Werteerfassung, Nachregulierung und Eichung, eines Gerätes zur Behandlung mit Kernspinresonanzen bereitzustellen, bei welchem sich die Geräteparameter in Abhängigkeit der Indikation auf einfachere und wirkungsvollere Weise genauer einstellen lassen.

### Zusammenfassung der Erfindung

Die Aufgabe der Erfindung wird bereits durch ein Verfahren zur Einstellung eines Gerätes zur Behandlung mit Kernspinresonanzen, durch ein Behandlungssystem sowie durch die Verwendung von Kernspinresonanzen nach einem der unabhängigen Ansprüche gelöst.

Bevorzugte Ausführungsformen und Weiterbildungen der Erfindung sind dem Gegenstand der abhängigen Ansprüche, der Beschreibung sowie den Zeichnungen zu entnehmen.

Die Erfindung betrifft ein Verfahren zur Einstellung, insbesondere der gezielten Kontrolle, Werteerfassung, Nachregulierung und insbesondere ein Verfahren zur Eichung, eines Gerätes zur Behandlung mit Kernspinresonanzen.

Die Erfindung bezieht sich insbesondere auf Geräte zur Erzeugung von Kernspinresonanzen, bei denen magnetische Felder mit einer Feldstärke von weniger als 50 Gauß, besonders bevorzugt von weniger als 30 Gauß, und Modulationsfrequenzen fₘ von weniger als 100 Hz erzeugt werden.

Die Frequenz des senkrecht eingestrahlten Wechselfeldes beträgt vorzugsweise weniger als 100 kHz und besonders bevorzugt weniger als 50 kHz.

Die Modulationsfrequenz fₘ liegt vorzugsweise zwischen 1 und 100 Hz.

Es wird der Einfluss der Behandlung mit Kernspinresonanzen auf die zelluläre Uhr eines Benutzers und/oder den Chronotyp eines Benutzers bestimmt und wobei basierend darauf das Gerät zur Behandlung mit Kernspinresonanzen eingestellt.

Wie weiter unten beschrieben wird, kann aber auch zunächst, vor dem Einstellen des Gerätes, eine Phase einer zellulären Uhr einer Zellkultur, eines Benutzers und/oder der Chronotyp eines Benutzers bestimmt werden.

Ein verfahrensbezogenes Ausführungsbeispiel der vorliegenden Erfindung betrifft ein Verfahren zur Einstellung, insbesondere Eichung, eines Gerätes zur Behandlung mit Kernspinresonanzen. Bei diesem Einstell-, insbesondere Eichverfahren, wird der Einfluss der Behandlung mit Kernspinresonanzen auf die zelluläre Uhr einer Zellkultur, eines Benutzers und/oder auf den Chronotyp eines Benutzers bestimmt. Basierend darauf wird das Gerät zur Behandlung mit Kernspinresonanzen eingestellt.

Wie weiter unten beschrieben wird, können zur Bestimmung des Einflusses der Behandlung mit Kernspinresonanzen auf die zirkadiane Uhr die Parameter, mit denen die Behandlung durchgeführt wird, variiert werden und in Abhängigkeit des Einflusses auf die zirkadiane Uhr die Parameter zur Behandlung eingestellt werden.

Es kann auch eine humane Zellkultur oder eine Zellkultur tierischen Ursprungs einer Behandlung mit Kernspinresonanzen ausgesetzt werden und es wird der Einfluss der Behandlung mit Kernspinresonanzen auf die zelluläre Uhr der Zellen der Zellkultur bestimmt.

Insbesondere können Zellkulturen humanen Ursprungs, wie beispielsweise Leberzellen oder Knorpelzellen verwendet werden.

Studien Im Bereich der Leberregeneration zeigen, dass durch Veränderung der Behandlungsparameter die Zellteilungsrate sowohl verlangsamt oder auch beschleunigt werden kann (siehe N. Budny, Spiegel et al.). Während der Zellteilung wurden Botenstoffe beobachtet die die Zellteilung und somit die Regeneration entscheidend beschleunigt können.

Es wird, um ein Gerät einzustellen, insbesondere zu eichen, der Einfluss der Behandlung mit Kernspinresonanzen auf die zirkadiane Uhr des jeweiligen Zelltyps bestimmt. Dafür werden die Parameter, mit denen die Behandlung durchgeführt wird, variiert und in Abhängigkeit des Einflusses auf die zirkadiane Uhr werden die Parameter zur Behandlung eingestellt.

Es ist auch bekannt, dass alle Licht lebenden Organsimen und damit auch der Mensch eine innere zirkadiane Uhr besitzen, die es auf zellulärer Ebene durch transkriptionelle und metabolische Prozesse ermöglicht, die Zeiterfassung ohne äußere Reize wie Licht über mehrere Tage beizubehalten. Diese zirkadiane Uhr steht in wechselseitiger Beziehung mit dem Hypoxie-Signalweg. Es ist bekannt, dass der Hypoxie-induzierte Faktor 1 (Hif-1α) unter anderem für die Expression von Erythropoetin verantwortlich ist, ebenso wie für den stark veränderten Stoffwechsel in Anpassung an geringe Sauerstoffkonzentrationen, hier ist vor allem der Glukosestoffwechsel zu erwähnen.

Darüber hinaus gibt es eine Vielzahl von Genen, die von Hif-1 reguliert werden und in vielen Schaltkreisen der Zelle eine Rolle spielen, wie beispielsweise bei der Zellvermehrung, der Apoptose oder der Gefäßneubildung.

Diesbezüglich wird insbesondere auf folgende Publikationen verwiesen:
Egg, M.; Paulitsch, M.; Ennemoser, Y.; Wüstenhagen, A.; Schwerte, T.; Sandbichler, A. M.; Fiechtner, B.; Köblitz, L.; Prem, C.; Pelster, B. (2014): Chronodisruption increases cardiovascular risk in zebrafish via reduced clearance of senescent erythrocytes.In: CHRONOBIOLOGY INTERNATIONAL 31/5, S. 680 - 689. (DOI).
Egg, M.; Köblitz, L.; Hirayama, J.; Schwerte, T.; Folterbauer, C.; Kurz, A.; Fiechtner, B.; Möst, M.; Salvenmoser, W.; Sassone- Corsi, P.; Pelster, B. (2013): Linking oxygen to time: The bidirectional interaction between the hypoxic signaling pathway and the circadian clock. In: CHRONOBIOLOGY INTERNATIONAL 30/4, S. 510 - 529.
Pelster, B.; Egg, M. (2015): Multiplicity of Hypoxia-Inducible Transcription Factors and Their Connection to the Circadian Clock in the Zebrafish. In: PHYSIOLOGICAL AND BIOCHEMICAL ZOOLOGY 88/2, S. 146 - 157. (DOI).
Sandbichler, A.M.: Jansen, B.; Peer, B.A..; Paulitsch, M.; Pelster, B.; Egg, M. (2018): Metabolic Plasticity Enables Circadian Adaptation to Acute Hypoxia in Zebrafish Cells. In: CELLULAR PHYSIOLOGY AND BIOCHEMISTRY 46/3, S. 1159 - 1174. (DOI)

Die zirkadiane Uhr ist bei Säugetieren und damit auch beim Menschen hierarchisch aufgebaut und umfasst eine Region, die sich im Hypothalamus befindet, den sogenannten Suprachiasmatischen Nukleus (SCN), sowie periphere Uhren, die organspezifisch und/oder gewebsspezifisch sind, und zelluläre Uhren, die sich in allen Zellen eines Organismus befinden und die gemeinsam mit den peripheren Uhren vom SCN koordiniert werden. Die Synchronisation der inneren Uhr mit dem äußeren Tag/Nachtrhythmus erfolgt dabei hauptsächlich über die Information von Lichtphotonen, die über die Retina des Auges in den SCN übertragen werden und dort sowohl neuronale als auch humorale Veränderungen bewirken. In allen Licht lebenden Organismen, also auch im Menschen, werden viele physiologische Prozesse zirkadian reguliert. Hierzu gehören insbesondere Körpertemperatur, Blutdruck, Hautwiderstand und Herzaktivität, sowie Hormonsekretion oder die Organspezifische Aktivität (die Aktivität der Leber, der Niere, des Herzens, etc. unterliegt jeweils einem spezifischen Tag/Nacht Rhythmus). Mit dem äußeren Tag/Nacht-Rhythmus synchronisierte zirkadiane Rhythmen stellen für den Organismus einen Vorteil dar und länger andauernde Störungen dieser Synchronisation (durch Schichtarbeit, Jetlag oder Schlafstörungen) begünstigen die Entstehung einer ganzen Reihe von Krankheiten, wie z.B. Herzkreislauferkrankungen, hormoninduzierte Tumoren, Diabetes, Fettleibigkeit, aber auch Schmerz- und Entzündungskrankheiten wie Arthrose.

Der vereinfachte molekulare Mechanismus der zellulären zirkadianen Uhr besteht aus mehreren Feedbackschleifen, von denen die zentrale Rückkoppelungsschleife im Folgenden beschrieben wird:
In der zentralen negativen Rückkoppelungsschleife dimerisieren das Hirn- und Muskel-Arnt-like-Protein (Bmal1) und das "Circadian locomotor output cycles kaput1" (Clock1) Protein und binden an E-box-Promoterelemente auf der DNS ihrer Gegenspieler, den Genen *Period* (*Per*1-3) und *Cryptochrom* (*cry*1/2). Infolgedessen erhöht sich die Expression der mRNA dieser Gene und damit auch die ihrer Proteinprodukte im Zytoplasma, wo beide Proteine sich aneinanderlagern und dimerisieren. Von dort aus wandern sie erneut in den Kern, wo der Protein-Komplex die Genexpression von *Clock*/*Bmal1* hemmt. Die negative Feedbackschleife wird mit der Phosphorylierung des PER/CRY-Komplexes durch Kaseinkinase Iε (CKIε) und durch die nachfolgende Degradation im Proteasom abgeschlossen.

Bekannte Vertreter dieser zentralen Rückkoppelungsschleife sind also die "Uhr-Gene" *clock1*, *bmal1*, *per1*, *per2*, *cry1* und *cry2*.

Auf molekularer Ebene eng verflochten mit der inneren Uhr ist der Hypoxie-Signalweg und zwar sowohl z.B. im Zebrafisch als auch bei Säugern. Da es sich also offensichtlich bei beiden Signalwegen um sehr konservierte Prozesse handelt, ist der Rückschluss auf eine bestehende Interaktion beider Signalwege auch beim Menschen zulässig. Der Hypoxie- Signalweg wiederum spielt ebenso wie die zirkadiane Uhr selbst bei zahlreichen humanen Erkrankungen eine zentrale Rolle. Hier sind vor allem Erkrankungen zu nennen, bei denen es zu einer verminderten Sauerstoffsättigung im Gewebe kommt, wie z.B. Herzinfarkt oder Schlaganfall oder aber auch Erkrankungen wo eine erniedrigte Sauerstoffsättigung unter normalen physiologischen Bedingungen benötigt wird wie z.B. in Gelenken. Hier wäre vor allem die Arthrose zu erwähnen, und tatsächlich wurde die Bedeutung des Hypoxie-Signalweges für die Entstehung und Progression von Arthrose bereits mehrfach untersucht und beschrieben.

Die biologisch dafür notwendigen Eiweiße, die den Zellstoffwechsel unter Sauerstoffmangel regulieren und koordinieren, sind Transkriptionsfaktoren aus der Gruppe der Hypoxieinduzierenden Faktoren (Hif), deren wichtigster Vertreter Hif-1α ist.

Beide Signalwege, die zirkadiane Uhr und der Hypoxie-Signalweg sind zudem von der verfügbaren Menge der freien Sauerstoffradikale (ROS) wechselseitig abhängig, die wiederum unter physiologischen Bedingungen sehr streng kompartimentiert und reguliert wird, und zwar durch verschiedenste enzymatische Sauerstoffradikalfänger (Antioxidatien) wie z.B. Katalase, Superoxiddismutase, Glutathionperoxidase oder aber auch durch die so genannten Peroxiredoxine (PRX), niedermolekulare Eiweiße, die selbst zirkadianen Reduktions- und Oxidationszyklen unterliegen. Die Rolle der freien Sauerstoffradikale (ROS) bei Entzündungsgeschehen und auch im natürlichen zellulären Alterungsprozess wurde bereits ausgiebig diskutiert und beschrieben und ist immer noch im Fokus aktueller Forschung.

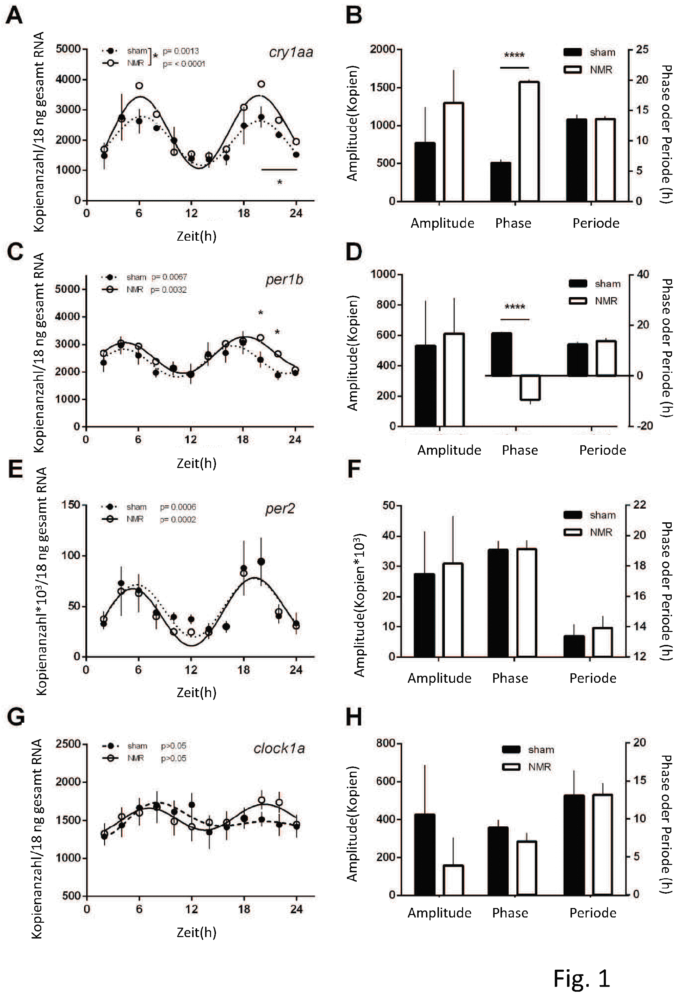

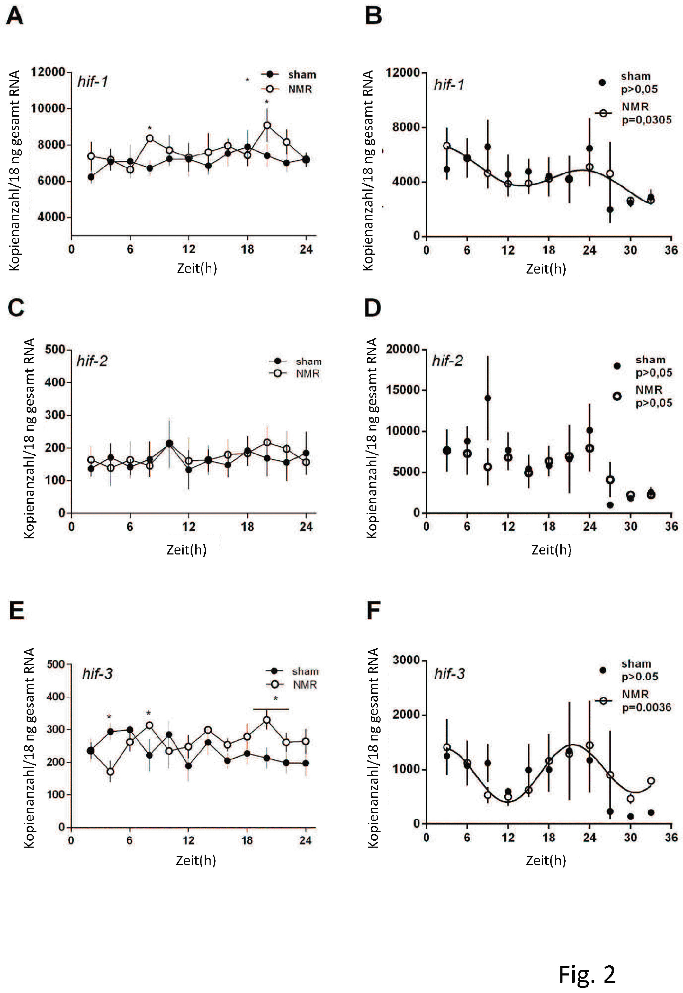

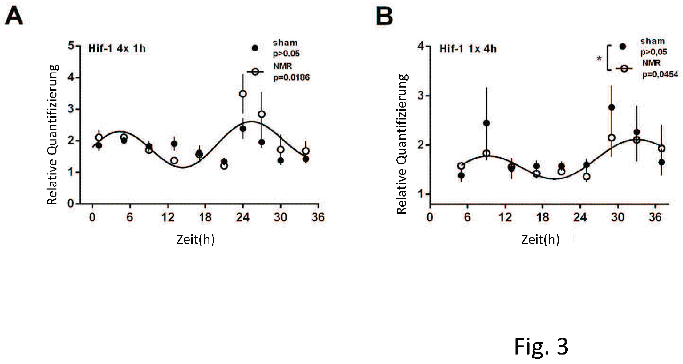

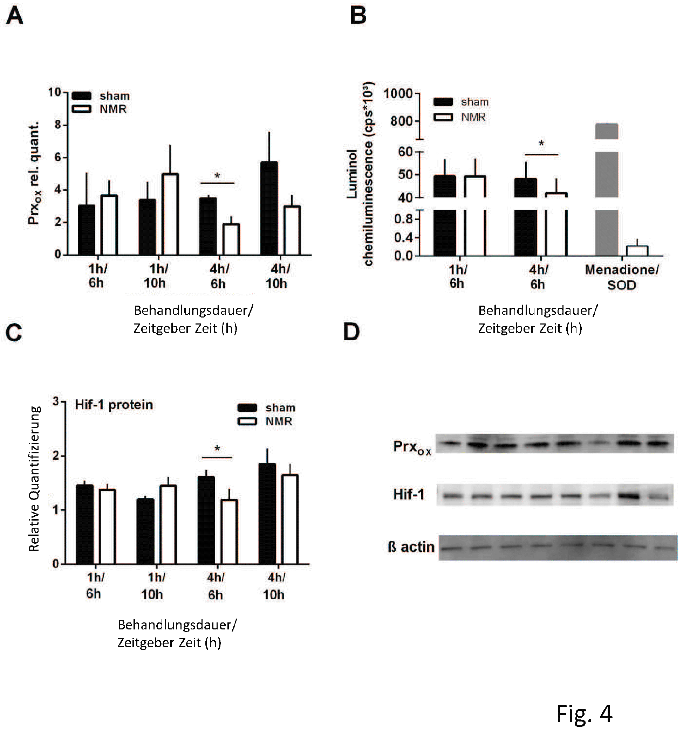

Es hat sich nun herausgestellt, dass die Oszillationen der zirkadianen Gene *period1* und *cryptochrome1* z.B. der Zebrafisch- Fibroblasten Zelllinie Z3 durch eine Behandlung mit Kernspinresonanzen signifikant in ihren Phasen verschoben werden. Diese Phasenverschiebung erfolgt dabei unter völligem Ausschluss von Licht (siehe Fig 1). Zudem wird die Expression von sauerstoffregulierenden Genen wie *hif-1α* und *hif-3α* signifikant auf mRNA Ebene in Zellen und in ganzen Zebrafischlarven (Fig.2, A, B, E und F) verändert, während die Isoform hif-2α überhaupt nicht beeinflusst wird (Fig.2, C und D), was wiederum auf die spezifische und selektive Wirkung der Kernspinresonanzen auf die Hif-Isoformen hinweist. Auf Hif-1α Proteinebene lässt sich in der Zellkultur ein klarer Synchronisierungseffekt der Kernspinresonanzen auf die zirkadiane Rhythmik des Proteins erkennen (Fig.3, A und B), der sowohl bei einer einmaligen 4-stündigen Behandlung also auch bei einer viermal wiederholten einstündigen Behandlung auftritt. Die Menge an oxidiertem Peroxiredoxin (Prx), an freien Sauerstoffradikalen und an Hif-1 Protein (Fig.4, A, B und C) werden darüber hinaus in einer Tageszeit und Dosis abhängigen Weise in die gleiche Richtung reguliert, in diesem Fall reduziert.

Aufgrund dieser neuen Kenntnisse ist es möglich, ein Gerät zur Erzielung von Kernspinresonanzen derart einzustellen und zu verwenden, dass sich ein verbesserter Behandlungserfolg erzielen lässt.

Beispielsweise mit einer Mikrocomputerchipkarte werden über einen Chipkartenleser die gespeicherten Behandlungssequenzen in das Steuergerät eingelesen und sorgen für den präzisen und ordnungsgemäßen Ablauf der Kernspinresonanzbehandlung.

Mehrere mögliche aufeinanderfolgende Behandlungszeitintervalle, die beispielsweise die auf der Chipkarte abgespeichert werden können, sorgen für die optimalen Steuerungsvorgaben für das Behandlungsgerät.

Die Sequenzen auf der Mikrocomputerchipkarte, der Behandlungsablauf und Behandlungszeit können wahlfrei verändert werden um die Resonanzeffekte während der Behandlungszeit auszulösen. Dies ist entscheidend für das Auslösen der Resynchronisation der zirkadianen Uhr der Zellen.

Über eine in die Behandlungseinheit eingebrachte Empfängerspule mit elektronischer Überwachungselektronik kann die ordnungsgemäße Funktion des Kernspinbehandlungsfeldes, die Behandlungsparameter, Veränderungen in der Feldstärke und Intensität, der in den Körper eingebrachten Energiedosis, der benötigten Resonanzbedingungen und sonstige vorbestimmte wichtige Prüfparameter optisch und akustisch überwacht und für Nachregulierungsprozesse der Qualität, der Effektivität und der Sicherheit genutzt werden.

Dieser Nachregulierung zur Optimierung der Behandlungsparameter kann über eine Rückmelde- oder Rückkopplungsschleife verwirklicht werden.

Eine weitere Überwachungselektronik misst und überwacht Temperaturveränderungen im Bereich der Haut, um Stoffwechselveränderungen zu erfassen und diese als Optimierungsparameter in den Regel- und Kontrollprozess der Steuerelektronik des Behandlungsablaufes einzubeziehen
Insbesondere lässt sich so das Gerät derart parametrieren und verwenden, dass es zu einer Synchronisierung der zellulären zirkadianen Uhr kommt. Dies verbessert die Wirksamkeit einer Behandlung, insbesondere die Wirksamkeit einer Behandlung von Arthrose-Patienten.

In Abhängigkeit von der jeweiligen Phase der zellulären Uhr wird insbesondere der Behandlungszeitpunkt, die Behandlungsdauer, das Behandlungsintervall, also der Abstand zwischen den Behandlungen, eine Modulationsfrequenz eines Feldes für einen adiabatischen Durchlauf und/oder eine Feldstärke eines von dem Gerät zur Behandlung mit magnetischen Feldern erzeugten Magnetfeldes eingestellt.

Insbesondere kann somit beispielsweise bei dem weiter oben genannten verfahrensbezogenen Ausführungsbeispiel, vorgesehen sein, dass in Abhängigkeit von der zellulären Uhr eine Behandlungsdauer, ein Behandlungsintervall, eine Tageszeit der Behandlung, eine Modulationsfrequenz eines Feldes und/oder eine Feldstärke eines von dem Gerät zur Behandlung mit magnetischen Feldern erzeugten Magnetfeldes eingestellt wird.

Des Weiteren kann insbesondere bei einem der bereits genannten verfahrensbezogenen Ausführungsbeispiele vorgesehen sein, dass das Gerät zur Behandlung mit Kernspinresonanzen derart eingestellt wird, dass ein Maximum oder ein Minimum einer spezifischen Proteinexpression und/oder eine maximale oder minimale Phasenverschiebung der zirkadianen Uhr, und/oder eine Synchronisation der zirkadianen Uhr erreicht werden.

Beim Behandlungsintervall ist gemäß einer Ausführungsform der Erfindung insbesondere vorgesehen, eine Behandlung immer in einem 12- oder 24 Stunden-Rhythmus +/- 2 Stunden, durchzuführen. Andere Behandlungsintervalle sind ebenso möglich.

Das hierfür verwendete Gerät hat vorzugsweise einen Speicher, in welchem sowohl Parametrisierungsdaten, wie Behandlungsdauer, Modulationsfrequenz fₘ und/oder Behandlungsintervall sowie Patientendaten hinterlegt sind.

Basierend hierauf kann in dem Gerät ein Behandlungsplan gespeichert werden, so dass das Gerät eine Behandlung nur dann ausführt, wenn diese entsprechend des gespeicherten Behandlungsplans durchgeführt wird.

Bei einem Verfahren nach einem der zuvor beschriebenen verfahrensbezogenen Ausführungsbeispiele kann zur Einstellung des Gerätes zur Behandlung mit Kernspinresonanzen der Chronotyp des Benutzers bestimmt werden und das Gerät in Abhängigkeit dieses Chronotyps eingestellt werden.

Zur Eichung des Gerätes wird die individuelle zirkadiane Uhr, also der Chronotyp des Benutzers berücksichtigt.

Grundsätzlich gibt es beim Menschen zwei Chronotypen (mit verlaufenden Formen dazwischen), den so genannten Frühaufsteher ("die Lärche") und den Spätaufsteher ("die Eule"), welche physiologisch bereits charakterisiert wurden.

Hierfür bietet sich insbesondere die Messung der Körpertemperatur, der Herzfrequenz, des Hautwiderstands und/oder des Blutdrucks über einen Zeitraum von vorzugsweise mindestens 24, besonders bevorzugt mindestens 48 Stunden an, um so die individuelle zirkadiane Uhr des Benutzers darzustellen.

Insbesondere bei dem zuvor genannten verfahrensbezogenen Ausführungsbeispiel, kann demnach beispielsweise vorgesehen sein, dass die zirkadiane Uhr durch Temperaturmessung, Blutdruckmessung, Messung des Hautwiderstands und/oder Bestimmung der Herzfrequenz ermittelt wird.

Basierend hierauf können insbesondere die Behandlungsintervalle und/oder der optimale Zeitpunkt der Behandlung bestimmt werden.

Die Erfindung betrifft des Weiteren ein Gerät zur Behandlung mit Kernspinresonanzen, welches mit dem vorstehend beschriebenen Verfahren eingestellt ist.

Ein entsprechendes Ausführungsbeispiel betrifft somit ein Gerät zur Behandlung mit Kernspinresonanzen, welches mit einem der voranstehend beschriebenen verfahrensbezogenen Ausführungsbeispiele eingestellt ist.

Wie bereits beschrieben und nachfolgend weiter ausgeführt wird, betrifft die Erfindung auch ein Gerät zur Behandlung mit Kernspinresonanzen, welches zum einen eingerichtet ist, magnetische Felder zur Erzeugung der Kernspinresonanzen zu erzeugen. Die dazu vorgesehenen Mittel können auch als Behandlungseinheit bezeichnet werden. Zum anderen, umfasst das Gerät zur Behandlung mit Kernspinresonanzen bevorzugt auch eine Gerätesteuerung zur Steuerung des Gerätes, wobei diese Gerätesteuerung dazu eingerichtet ist, das Gerät in Abhängigkeit einer Phase einer zellulären Uhr und/oder eines Chronotyps eines Benutzers einzustellen.

Die Erfindung betrifft des Weiteren ein Behandlungssystem mit einem Gerät zur Behandlung mit Kernspinresonanzen. Das Behandlungssystem weist Mittel, insbesondere einen Sensor, zur Bestimmung des Chronotyps eines Benutzers auf.

Mit anderen Worten betrifft ein systembezogenes Ausführungsbeispiel ein Behandlungssystem mit einem Gerät zur Behandlung mit Kernspinresonanzen sowie mit Mitteln, insbesondere einem Sensor, zur Bestimmung des Chronotyps eines Benutzers. Das Gerät kann dabei insbesondere gemäß einem der voranstehend beschriebenen verfahrensbezogenen Ausführungsbeispiele eingestellt sein.

Der Sensor ist gemäß einer Ausführungsform der Erfindung zur Messung der Körpertemperatur, des Blutdrucks, des Hautwiderstands und/oder der Herzfrequenz eines Benutzers ausgebildet.

Das heißt, dass beispielsweise bei dem zuvor beschriebenen systembezogenen Ausführungsbeispiel der Sensor zur Messung der Körpertemperatur, des Blutdrucks, des Hautwiderstands und/oder der Herzfrequenz eines Benutzers ausgebildet sein kann.

Bei einer Ausführungsform der Erfindung ist ein kombinierter Sensor vorhergesehen, der über einen Zeitraum von mindestens 24, vorzugsweise mindestens 48 Stunden Körpertemperatur, Hautwiderstand und Herzfrequenz misst.

Bei einer anderen Ausführungsform wird, vorzugsweise allein, der Hautwiderstand über den genannten Zeitraum gemessen. Diesbezüglich wird auf Vidacek et al: Personality differences in the phase of the circadian rhythms: a comparison of morningness and extraversion, ERGONOMICS 1988, Vol. 31, No. 6, 873-888 verwiesen. Es ist demnach bekannt, dass allein der Hautwiderstand für eine Bestimmung des Chronotyps verwendet werden kann.

Die Berechnung des Chronotyps auf Basis der gemessenen Werte kann in einer externen Einheit, in welcher der Sensor angeordnet ist, in der Gerätesteuerung des Gerätes zur Erzeugung von Kernspinresonanzen oder in einer sonstigen externen Recheneinheit erfolgen.

Entscheidend ist, dass der Gerätesteuerung des Gerätes zur Erzeugung von Kernspinresonanzen Informationen über den Chronotyp des Benutzers zur Verfügung gestellt werden.

Gemäß einer Ausführungsform überträgt der Sensor Daten drahtlos an eine Gerätesteuerung.

Mit anderen Worten kann insbesondere bei einem System entsprechend einem der beschriebenen systembezogenen Ausführungsbeispiele der Sensor des Weiteren ausgebildet sein, Daten, vorzugsweise drahtlos, an eine Gerätesteuerung zu übertragen.

Gemäß einer anderen Ausführungsform werden die in einer externen Einheit, die den Sensor umfasst, gemessenen Daten gespeichert und durch Anschließen der externen Einheit, insbesondere an eine Gerätesteuerung, übertragen.

Entsprechend kann in einem Behandlungssystem nach einem der bereits beschriebenen systembezogenen Ausführungsbeispiele der Sensor des Weiteren Teil einer externen Einheit sein.

Die Informationen zum Chronotyp der Benutzer können auf einem externen Speicher, aber auch in einem Speicher der Gerätesteuerung des Gerätes zur Behandlung mit Kernspin-resonanzen hinterlegt sein.

Das Gerät zur Erzeugung von Kernspinresonanzen wird vorzugsweise über einen herausnehmbaren Speicher, insbesondere über eine komplexe Mikroprozessor-Chipkarte mit Speicherbereich, gesteuert. Durch diese werden über mehrere zeitlich veränderbare programmierbare Programmstufen die biologischen Prozesse der Retriggerung der zirkadianen Uhr ausgelöst.

Der Behandlungsablauf ist vorzugsweise automatisiert und verläuft über mehrere zeitlich festgelegte Programmstufen.

Die Behandlungsdaten und Behandlungszeiten werden vorzugsweise im Steuergerät zwischengespeichert um bei Stromausfall die Behandlung ohne Behandlungslücken oder Verfälschung fortführen zu können.

In einer anderen Ausführungsform werden die biologischen und elektrischen Patientendaten über Sensoren vor der Therapie am Patienten gemessen, gespeichert, anschließend direkt für den Behandlungsablauf als Referenz- oder Behandlungsvorgabe genutzt oder vor Therapiebeginn in das Steuergerät eingelesen.

Bei der Einstellung des Gerätes kann über die Daten des Sensors die individuelle zirkadiane Uhr des Benutzers berücksichtigt werden. Insbesondere können Behandlungszeit, Behandlungsdauer, Behandlungsintervalle und/oder die Modulationsfrequenz fₘ in Abhängigkeit der übermittelten Daten des Sensors bestimmt werden.

Die Erfindung betrifft die Verwendung von Kernspinresonanzen zur Synchronisierung und/oder Neutaktung der zirkadianen Uhr der Zellen eines Benutzers. Es hat sich herausgestellt, dass insbesondere die *period*1 und *cryptochrome1* mRNA Oszillationen durch eine gezielte Kernspinresonanzbehandlung in ihren Phasen verschoben werden und die zirkadiane Uhr dadurch synchronisiert werden kann. Des Weiteren wird auch die zirkadiane Oszillation von Hif-1α Protein auf Zellebene durch Kernspinresonanzen synchronisiert. Zudem können Zeit und Dosis abhängige Veränderungen bei Hif-1, Prx und der Menge an freien Sauerstoffradikalen erzeugt werden, deren Bedeutung für die humane Pathophysiologie bereits erwähnt wurden (siehe oben).

Ein verwendungsbezogenes Ausführungsbeispiel betrifft demnach insbesondere die Verwendung von Kernspinresonanzen zur Veränderung, insbesondere Synchronisation, der zirkadianen Uhr der Zellen eines Benutzers.

Die erfindungsgemäße Verwendung von Kernspinresonanzen bezieht sich auf eine ausschließlich kosmetische Behandlung, insbesondere die Behandlung von Cellulite.

Mit anderen Worten wird insbesondere bei dem vorstehend genannten verwendungsbezogenen Ausführungsbeispiel die zirkadiane Uhr zu kosmetischen Zwecken verändert.

Bei einem der verwendungsbezogenen Ausführungsbeispiele kann des Weiteren vorgesehen sein, dass der Hypoxie-Signalweg von Zellen des Benutzers verändert wird.

Außerdem kann bei einer Verwendung entsprechend einem der genannten Verwendungs-Ausführungsbeispiele vorgesehen sein, dass die Menge an freien Sauerstoffradikalen von Zellen des Benutzers verändert wird.

Sehr effektiv und hochwirksam lässt sich das Kernspinresonanzverfahren in allen Bereichen gestörter oder degenerativer Zellfunktionen im Bereich der Gelenke des Knochens, der Organe, Bänder, Muskel, Sehnen sowohl in der nachoperativen Wundheilung, Hautverbrennungen, der Stoffwechsel- bzw. Durchblutungsstörung im Bereich des Knochens, der Frakturheilung, etc. als auch in anderen regenerationsfähigen Bereichen einsetzen.

Je nach gewünschtem Zweck werden gemäß der Erfindung die Parameter der Behandlung mit Kernspinresonanzen, insbesondere der Behandlungszeitpunkt, das Behandlungsintervall, die Behandlungsdauer und/oder die Modulationsfrequenz fₘ derart eingestellt bzw. eingelesen, dass sich eine maximale Phasenverschiebung oder eine minimale Phasenverschiebung, ergibt, bzw. eine bestmögliche Synchronisation der zirkadianen Uhr.

### Kurzbeschreibung der Zeichnungen

Der Gegenstand der Erfindung soll im Folgenden bezugnehmend auf die Zeichnungen Fig. 1 bis Fig. 5 näher erläutert werden.
In Fig. 1 sind die über die Zeit aufgetragenen mRNA Kopienzahlen representativer Gene aus der zentralen Rückkoppelungsschleife der Inneren Uhr dargestellt, die die Oszillation der Inneren Uhr wiedergeben. Gezeigt werden die Oszillationen von *cryptochrome1*, *period1*, *period2* und *clock1*. Zu sehen ist der Vergleich der Schwingungen zwischen scheinbehandelten Zellen und solchen, die an vier aufeinanderfolgenden Tagen zur gleichen Zeit für jeweils eine Stunde mit Kernspinresonanzen behandelt wurden. Deutlich erkennt man die Verschiebung der Phase, die bei den Genen *period1* und *cryptochrome1* signifikant ist (cosinor fit analysis, GraphPad Prism 6.0)
Fig.2 zeigt die Veränderungen der mRNA Konzentrationen von *hif-1α* und *hif-3α* aus dem Hypoxie-Signalweg zwischen scheinbehandelten Zellen und solchen, die an vier aufeinanderfolgenden Tagen zur gleichen Zeit für jeweils eine Stunde mit Kernspinresonanzen behandelt wurden (A und E), bzw. die selektive Synchronisierung der mRNA- Oszillationen von *hif-1α* und *hif-3α* in ganzen Zebrafischlarven nach gleichem Behandlungsschema (B und F), während *hif-2α* sowohl in Zellen als auch in ganzen Zebrafischlarven unbeeinflusst bleibt (Fig.2, C und D).
Fig.3 zeigt die durch Kernspinresonanzen erzeugte Synchronisierung der zirkadianen Hif-1α Protein Oszillationen in Zebrafischzellen, einmal nach einer einstündigen Behandlung, die viermal wiederholt wurde (A), bzw. nach einer einmaligen vierstündigen Behandlung (B).
Fig.4 zeigt die verringerten und dosisabhängigen Mengen an oxidiertem Peroxiredoxin (A), freien Sauerstoffradikalen (B) und Hif-1α Protein (C). Eine 4-stündige Behandlung führt im Vergleich zu einer nur einstündigen Behandlung zu einer Reduktion der Proteinmengen, bzw. der Menge an freien Sauerstoffradikalen.
Fig. 5 ist ein schematisches Flussdiagramm eines Verfahrens zur Einstellung eines Gerätes zur Behandlung mit Kernspinresonanzen.
Fig. 6 ist eine schematische Darstellung eines Gerätes zur Behandlung mit Kernspinresonanzen.

### Detaillierte Beschreibung der Zeichnungen

Bezugnehmend auf die Grafiken gemäß Fig. 1 bis Fig. 6 soll der Einfluss einer Behandlung mit Kernspinresonanzen auf verschiedene biologische Parameter erläutert werden.

In Fig. 1 und 2 ist jeweils auf der x-Achse die Tageszeit und auf der y-Achse die spezifische mRNA Kopienanzahl bezogen auf 16 ng Gesamt-RNA aufgetragen. Verglichen wird jeweils eine scheinbehandelte Kontrollgruppe sowie eine Zellkultur, welche an vier aufeinanderfolgenden Tagen jeweils für eine Stunde mit einem MBST^{®}-Therapiegerät behandelt wurde. Die Behandlung erfolgte dabei jeweils zu der gleichen Tageszeit.

Hierzu wurde eine Zellkultur aus Zebrafisch-Fibroblasten verwendet.

In Fig. 1 sind die mRNA Oszillationen der Gen*e cryptochrome1, period1, period2* und *clock1* dargestellt.

Zu erkennen ist, dass gegenüber den scheinbehandelten Kontrollzellen die zirkadiane mRNA Oszillation der Gene *period1* und *cry1* um ca. zwei Stunden verschoben ist. Es ist mithin gelungen, die Phasen dieser Gen- Oszillationen ohne die äußere Einwirkung von Licht zu verschieben. Es liegt ein signifikanter Unterschied der Behandlungsgruppen vor, der über eine Cosinuskurven- Anpassung (Graphpad Prism 6) statistisch belegt wurde.

Fig. 2 zeigt die mRNA Mengen der Gene *hif-1α, hif-2α* und *hif-3α* im Tagesverlauf.

Zu erkennen ist, dass die Isoformen *hif-1α* und *hif-3α* durch Kernspinresonanzen selektiv angesteuert werden, auf Zellebene (A und E) und im gesamten Organismus (B und F), während *hif-2α* sowohl in Zellen als auch in ganzen Zebrafischlarven unbeeinflusst bleibt.

Fig. 3 zeigt die Hif-1α Proteinmengen von Zellen im Tagesverlauf. Zu erkennen ist, dass Kernspinresonanzen bei beiden Behandlungsvarianten (viermal eine Stunde [A]- im Vergleich zu einer einmaligen Behandlung für vier Stunden [B]) zu einer Synchronisierung der zirkadianen Rhythmik der Hif-1α Proteinexpression führt.

Fig. 4zeigt eine Abhängigkeit der Kernspinresonanzen- Wirkung von der Dosis: nur eine 4 stündige einmalige Behandlung zeigt eine signifikante Reduktion der Mengen an oxidiertem Prx (A), freien Sauerstoffradikalen (B) und Hif-1α (C)im Gegensatz zu einer Behandlung von nur einmal einer Stunde.

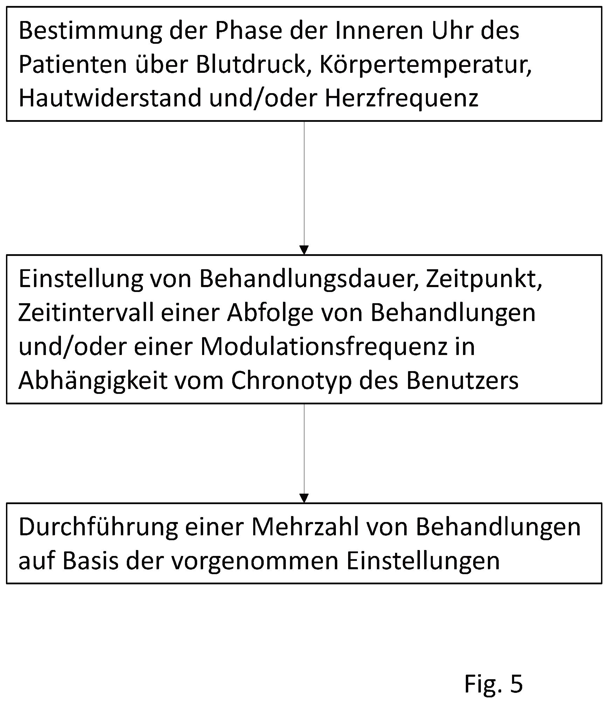

Fig. 5 ist ein Flussdiagramm eines Verfahrens zur Einstellung eines Gerätes zur Behandlung mit Kernspinresonanzen gemäß eines Ausführungsbeispieles der Erfindung.

Zunächst wird die Phase der Inneren Uhr des Patienten über Hautwiderstand, Blutdruck, Körpertemperatur und/oder Herzfrequenz bestimmt, also der individuelle Chronotyp ermittelt.

Basierend darauf wird Behandlungsdauer, Zeitintervall einer Abfolge von Behandlungen, Tageszeitfenster der Behandlung und/oder Modulationsfrequenz fₘ des Gerätes zur Behandlung mit Kernspinresonanzen in Abhängigkeit vom Chronotyp eingestellt.

Basierend auf dieser Einstellung wird eine Mehrzahl von Behandlungen vorgenommen. Es kann sich dabei sowohl um therapeutische, als auch kosmetische Behandlungen handeln.

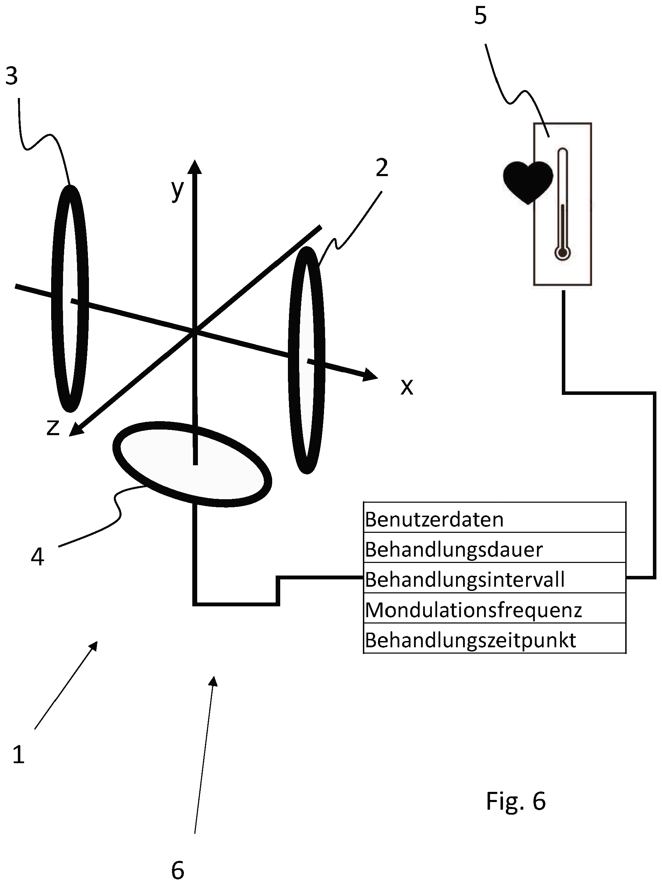

Fig. 6 ist eine schematische Darstellung eines erfindungsgemäßen Systems 6 zur Behandlung eines Benutzers mit Kernspinresonanzen. Das System 6 umfasst ein Gerät 1 zur Erzeugung von Kernspinresonanzen in einem zu behandelnden Gewebe.

Das hier schematisch dargestellte Gerät 1 umfasst die Spulen 2 und 3, welche sich in Helmholtz-Anordnung gegenüberstehen und mittels derer ein homogenes Magnetfeld erzeugt wird, welches sich entlang der Richtung der eingezeichneten x-Achse erstreckt.

Um eine Kernspinresonanz zu erzeugen, wird mittels der Spule 4 senkrecht zu dem von den Spulen 2 und 3 erzeugten homogenen Magnetfeld ein magnetisches Wechselfeld eingestrahlt.

Dessen Frequenz ist so abgestimmt, dass die Larmor-Bedingung erreicht wird.

Weiter setzt sich das Feld des von den Spulen 2 und 3 erzeugten homogenen Feldes aus einem Sockelbetrag und einem Modulationsbetrag zusammen, der mittels der Modulationsfrequenz fₘ, beispielsweise in Rechteckform, moduliert wird.

Durch die Modulation wird im Behandlungsvolumen bei jedem Durchlauf zumindest einmal die Resonanzbedingung erzeugt.

Das Gerät 1 umfasst einen Speicher, in welchem Benutzerdaten, Daten zur Behandlungsdauer, zum Behandlungsintervall und zur Modulationsfrequenz fₘ hinterlegt sind.

Behandlungszeitfenster, Behandlungsdauer, Behandlungsintervall und/oder Modulationsfrequenz werden in Abhängigkeit vom Chronotyp der zu behandelnden Person durchgeführt.

Erfindungsgemäß umfasst das System 6 Mittel zur Bestimmung des Chronotyps des Benutzers.

Im vorliegenden Fall handelt es sich dabei um einen Sensor 5, welcher Teil einer externen Einheit ist, welche am Benutzer angebracht werden kann und, vorzugsweise drahtlos, Daten an eine Gerätesteuerung des Geräts 1 überträgt. Dies kann beispielsweise auch über das Internet erfolgen.

Der Sensor 5 kann beispielsweise Herzfrequenz, Körpertemperatur oder Hautwiderstand über einen Zeitraum von mindestens 24 Stunden messen. Basierend auf den Messwerten wird der Chronotyp des jeweiligen Benutzers bestimmt.

In Abhängigkeit von der Phase der zirkadianen Uhr des Benutzers kann nunmehr, vorzugsweise automatisiert über die Gerätesteuerung, ein Behandlungsprofil mit optimierten Behandlungsparametern erstellt werden.

Dies gilt insbesondere für die Tageszeit, bei welcher die Behandlung erfolgen soll.

Das Gerät 1 umfasst vorzugsweise eine Gerätesteuerung, welche auch den Speicher mit den genannten Daten umfasst, wobei die Gerätesteuerung vorzugsweise automatisiert, beispielsweise auf Basis eines Berechnungsprogramms oder auf Basis einer Datenbank, optimierte Behandlungsparameter errechnet und, den jeweiligen Benutzerdaten zugeordnet, in dem Speicher hinterlegt.

Durch die hierauf basierende Einstellung der Parameter des Behandlungsgerätes kann die Wirksamkeit der Behandlung mit Kernspinresonanzen auf einfache Weise verbessert werden.

Über eine Mikrocomputerchipkarte werden über einen Chipkartenleser die gespeicherten Behandlungssequenzen in das Steuergerät eingelesen und sorgen für den gezielten und ordnungsgemäßen Ablauf der MBST^{®}-Kernspinresonanz-behandlung.

Die Sequenzen auf der Mikrocomputerchipkarte, der Behandlungsablauf und Behandlungszeit können wahlfrei verändert, kontrolliert und nachreguliert werden um die Resonanzeffekte während der Behandlungszeit auszulösen.

Dies ist entscheidend für die hochsignifikante Steigerung der ATP und des Hypoxiegehaltes der Zellen sowie für das Auslösen der Resynchronisation der Zirkadianen Uhr der Zellen.

Untersuchungen haben gezeigt, dass der Bereich des Immunsystems zu einer verstärkten Abwehrhaltung gebracht werden kann und entartete Zellfunktionen dadurch eliminiert werden können.

Diese Anmeldung ist eine Teilanmeldung der Anmeldung 18 739 793.0 (EP 3 648 840), die hiermit in ihrer Gesamtheit durch Referenz inkorporiert wird. Der Anmelder behält sich insbesondere das Recht vor, auf alle in der Beschreibung, den Ansprüchen und/oder Zeichnungen oder anderweitig in der Anmeldung 18 739 793.0 (EP 3 648 840) offenbarten Gegenstände in dieser Teilanmeldung zurückzukommen und/oder diese zu beanspruchen.

## Patentansprüche

1. Verfahren zur Einstellung eines Gerätes zur Behandlung mit Kernspinresonanzen,
wobei eine Phase einer zellulären Uhr einer Zellkultur, eines Benutzers und/oder der Chronotyp eines Benutzers bestimmt wird, und wobei basierend darauf das Gerät zur Behandlung mit Kernspinresonanzen eingestellt wird.

2. Verfahren nach dem vorstehenden Anspruch, wobei vor dem Einstellen des Gerätes der Einfluss der Behandlung mit Kernspinresonanzen auf die zelluläre Uhr einer Zellkultur, eines Benutzers und/oder auf den Chronotyp eines Benutzers bestimmt wird, und
wobei zur Bestimmung des Einflusses der Behandlung mit Kernspinresonanzen auf die zelluläre Uhr einer Zellkultur, eines Benutzers und/oder den Chronotyp eines Benutzers vorzugsweise die Behandlungsparameter variiert werden, und in Abhängigkeit des Einflusses auf die zelluläre Uhr die Parameter zur Behandlung eingestellt werden.

3. Verfahren nach dem vorstehenden Anspruch, wobei die Phase oder der Einfluss auf die zelluläre Uhr und/oder der Chronotyp mittels eines Sensors bestimmt wird, wobei der Sensor vorzugsweise zur Messung der Körpertemperatur, des Blutdrucks, des Hautwiderstands und/oder der Herzfrequenz eines Benutzers ausgebildet ist.

4. Verfahren nach einem der vorstehenden Ansprüche, wobei die Einstellung des Gerätes umfasst, dass eine Behandlungsdauer, ein Behandlungsintervall und/oder eine Tageszeit der Behandlung eingestellt wird.

5. Verfahren nach einem der vorstehenden Ansprüche, wobei das Gerät zur Behandlung mit Kernspinresonanzen dazu eingerichtet ist, magnetische Felder zu erzeugen, und wobei die Einstellung des Gerätes umfasst, dass eine Modulationsfrequenz und/oder eine Feldstärke der magnetischen Felder eingestellt wird.

6. Gerät zur Behandlung mit Kernspinresonanzen, umfassend eine Behandlungseinheit, welche dazu eingerichtet ist, magnetische Felder zur Erzeugung von Kernspinresonanzen zu erzeugen, und eine
Gerätesteuerung, welche dazu eingerichtet ist, das Gerät zu steuern,
**dadurch gekennzeichnet, dass** die Gerätesteuerung dazu eingerichtet ist, das Gerät in Abhängigkeit einer Phase einer zellulären Uhr und/oder eines Chronotyps eines Benutzers einzustellen.

7. Gerät zur Behandlung mit Kernspinresonanzen nach dem vorstehenden Anspruch, **dadurch gekennzeichnet, dass** die Gerätesteuerung dazu eingerichtet ist, als Einstellung des Gerätes eine Behandlungsdauer, ein Behandlungsintervall, eine Tageszeit der Behandlung, eine Modulationsfrequenz und/oder eine Feldstärke der magnetischen Felder festzulegen.

8. Gerät zur Behandlung mit Kernspinresonanzen nach einem der vorstehenden Ansprüche, **dadurch gekennzeichnet, dass** die Gerätesteuerung dazu eingerichtet ist, Daten zur Einstellung des Gerätes zu empfangen, indem die Daten von einem Datenträger eingelesen werden oder von einer externen Einheit, vorzugsweise drahtlos, an die Gerätesteuerung übertragen werden.

9. Gerät zur Behandlung mit Kernspinresonanzen nach einem der vorstehenden Ansprüche, ferner umfassend zumindest einen Speicher zur Hinterlegung von Daten zur Einstellung des Gerätes, wobei in dem zumindest einen Speicher insbesondere die Phase der zellulären Uhr und/oder der Chronotyp des Benutzers hinterlegt ist.

10. Gerät zur Behandlung mit Kernspinresonanzen nach dem vorstehenden Anspruch, wobei der zumindest eine Speicher einen in der Gerätesteuerung des Gerätes angeordneten Speicher umfasst oder einen externen Speicher umfasst.

11. Gerät zur Behandlung mit Kernspinresonanzen nach einem der vorstehenden Ansprüche, **dadurch gekennzeichnet, dass** die Behandlungseinheit dazu eingerichtet ist, als magnetische Felder zur Erzeugung der Kernspinresonanzen ein homogenes Magnetfeld sowie ein damit überlagertes
Wechselfeld zu erzeugen.

12. Gerät zur Behandlung mit Kernspinresonanzen, insbesondere nach einem der vorstehenden Ansprüche, **dadurch gekennzeichnet, dass** das Gerät eingerichtet ist, die zirkadiane Uhr, den Hypoxie-Signalweg und/oder die Menge an freien Sauerstoffradikalen von Zellen eines Benutzers zu verändern.

13. Behandlungssystem mit einem Gerät zur Behandlung mit Kernspinresonanzen, insbesondere einem Gerät nach einem der vorstehenden Ansprüche, **dadurch gekennzeichnet, dass** das Behandlungssystem Mittel, insbesondere einen Sensor, zur Bestimmung des Chronotyps eines Benutzers aufweist.

14. Behandlungssystem nach dem vorstehenden Anspruch, **dadurch gekennzeichnet, dass** der Sensor zur Messung der Körpertemperatur, des Blutdrucks, des Hautwiderstands und/oder der Herzfrequenz eines Benutzers ausgebildet ist und wobei die Mittel zur Bestimmung des Chronotyps vorzugsweise ferner eine Einrichtung zur Berechnung des Chronotyps auf Basis der gemessenen Werte umfasst.

15. Behandlungssystem nach einem der vorstehenden Ansprüche, **dadurch gekennzeichnet, dass** der Sensor ausgebildet ist, Daten, vorzugsweise drahtlos, an eine Gerätesteuerung zu übertragen und/oder dass der Sensor Teil einer externen Einheit ist.

16. Verwendung von Kernspinresonanzen zur Veränderung, insbesondere Synchronisation, der zirkadianen Uhr der Zellen eines Benutzers zu nicht-therapeutischen Zwecken.

17. Verwendung nach dem vorstehenden Anspruch, **dadurch gekennzeichnet, dass** der Hypoxie-Signalweg und/oder die Menge an freien Sauerstoffradikalen von Zellen des Benutzers verändert wird.
